# EUROPEAN PATENT SPECIFICATION

(11) **EP 1 776 128 B1**
(45) Date of publication and mention of the grant of the patent: **14.02.2018**
(21) Application number: 05753109.7
(22) Date of filing: 16.06.2005
(51) Int. Cl.: A61K 36/324

(54) **COMPOSITIONS FOR TOPICAL TREATMENT**
ZUSAMMENSETZUNGEN FÜR TOPISCHE BEHANDLUNG
COMPOSITIONS DE TRAITEMENT PAR VOIE TOPIQUE

(30) Priority: 22.06.2004 GB 0413954
(43) Date of publication of application: 25.04.2007
(73) Proprietor: Altunkaya, Ali, Adana (TR)
(72) Inventor: Altunkaya, Ali, Adana (TR)
(74) Representative: Evans, Jacqueline Gail Victoria
(86) International application number: PCT/IB2005/001692
(87) International publication number: WO 2006/000867

(56) References cited:
- EP-A- 0 552 657
- EP-A- 1 165 107
- WO-A-00/21381
- WO-A-91/11105
- WO-A-02/085921
- WO-A2-03/065980
- DATABASE WPI Section Ch, Week 199544 Derwent Publications Ltd., London, GB; Class B04, AN 1995-340208 XP002349172 & JP 07 233086 A (MORINAGA MILK IND CO LTD) 5 September 1995 (1995-09-05)
- DATABASE WPI Section Ch, Week 200341, Derwent Publications Ltd., London, GB; Class A96, AN 2003-437457 & NL 1 018 421 C (SARA LEE DE NV) 07 January 2003
- DATABASE EMBASE [Online] ELSEVIER SCIENCE PUBLISHERS, AMSTERDAM, NL 1998 Database accession no. EMB-1999237393 & BERARDESCA E.; BORRONI G.: "Oral and topical supplementation of linoleic acid and skin disease", MEDICINE BIOLOGIE ENVIRONEMENT, vol. 26, no. 2, 1998, pages 159-163,
- DATABASE EMBASE [Online] ELSEVIER SCIENCE PUBLISHERS, AMSTERDAM, NL 2002 Database accession no. emb-2003140238 & LASSUS A. ET AL: "Chitosan conjugated CLA gel for treatment of stable chronic psoriasis vulgaris", JOURNAL OF APPLIED COSMETOLOGY, vol. 20, no. 4, October 2002 (2002-10), pages 219-225,
- DATABASE EMBASE [Online] ELSEVIER SCIENCE PUBLISHERS, AMSTERDAM, NL 1953 Database accession no. EMB-0008260350 & RICCIARDI L.: "Concentrated linoleic acid in the treatment of psoriasis", , vol. 4, no. 2, 1953, pages 36-43,
- DATABASE WPI Section Ch, Week 200353, Derwent Publications Ltd., London, GB; Class B04, AN 2003-561912 & JP 2003 089629 A (KAKUNAI JUYOTAI KENKYUSHO) 28 March 2003

## Description

This invention relates to compositions for topical treatment of mammalian skin and particularly human skin.

Persistent skin diseases exist which are extremely resistant to remedial treatment. In particular eczema and psoriasis, among other skin diseases such as impetigo, Pemphigus Vulgaris, Roscicea folliculitis are responsible for considerable patient discomfort and disfigurement. Remedial treatments involving diet and topical application of medication provide relief in a few cases but little long term improvement.

Compositions comprising lactoperoxidase for the treatment of eczema or psoriasis are described in JP 07233086A and DATABASE EMBASE , database accession no. Emb-2003140238, 2002 (Elsevier Science Publishers). Antimicrobial compositions comprising glucose oxidase and lactoperoxidase for use as cosmetic and pharmaceutical compositions are described in EP-A-0552657

Linolenic acid has been described as being effective in the treatment of psoriatic lesions (see DATABASE WPI DERWENT PUBLICATIONS, 2003 database accession no. 2003-561912; DATABASE EMBASE , database accession no. Emb-1999237393, 1998 and database accession no. Emb-2003140238, Elsevier Science Publishers).

Boswellia extracts for application to irritated skin are described in EP-A-1165107 and the use of boswellic acids for the treatment of psoriatic lesions is described in EP-A-0552657.

Compositions comprising two antimicrobial enzymes and a polyunsaturated fatty acid such as arachidonic acid are described in WO 00/21381A.

Creams comprising an antibacterial enzyme system in an amollient base are described in WO 03/065980 A and DATABASE WPI DERWENT PUBLICATIONS, 2003 database accession no. 2003-437457.

It is principle advantage of the present invention to provide compositions for treatment of psoriasis, eczema and other undesirable skin disorders which are of relatively simple and inexpensive design and operation. The compositions are free from undesirable side effects, effectively treat the symptoms and can provide a cure for undesirable skin diseases.

A further advantage of the present invention is the provision of a method for treatment of psoriasis or other undesirable skin disorder which reduces and/or eliminates "itchiness" of skin which is a common symptom/condition of such skin disorders.

The present invention provides compositions for topical application to skin surfaces which relieve the distressing symptoms of the above afflictions and provide, in may cases, a long term cure.

According to the present invention there is provided the use of a composition comprising, in combination, one or more boswellic acids and an enzyme based antibacterial system comprising lactoperoxidase and glucose oxidase in the manufacture of a medicament for the treatment of eczema and psoriasis.

Also provided is a composition comprising, in combination, one or more boswellic acids and an enzyme based antibacterial system comprising lactoperoxidase and glucose oxidase for use in the treatment of eczema and psoriasis.

There is also provided a composition comprising, in combination, a fatty acid having a chain length of at least 18 carbon atoms and which contains at least two unsaturated linkages and an enzyme based antibacterial system comprising lactoperoxidase and glucose oxidase for use in the treatment of psoriasis, as well as the use of such a composition in the manufacture of a medicament for the treatment of psoriasis.

There is further provided a composition comprising, in combination, one or more boswellic acids, a fatty acid having a chain length of at least 18 carbon atoms and which contains at least two unsaturated linkages and an enzyme based antibacterial system comprising lactoperoxidase and glucose oxidase for use in the treatment of eczema and psoriasis, as well as the use of such a composition in the manufacture of a medicament for the treatment of eczema and psoriasis.

The enzyme based antibacterial system is preferably a mixture of lactoperoxidase, glucose oxidase, buffers and substrates that convert oxygen into oxidative compounds having antibacterial action. The antibacterial agent selectively inhibits high growth keratinocyte cells yet not affecting normal growth of cells. Preferably glucose is present as a substrate and the mixture is buffered to a pH in the range 5 to 6.5, most preferably in the range 5.3 to 5.6. The enzyme based antibacterial system is preferably activated by admixture with a combination of potassium iodide and sodium thiocyanate.

The fatty acid or combination of fatty acids are either saturated or unsaturated and containing 8 to 28 carbon atoms. Preferably the composition is in the form of a cream emulsion for topical application containing from 5 to 40% by weight of caprylic capric triglycerides, sunflower, safflower, soybean, corn, evening primrose, refined herring or tuna oil and other oil as carrier. It may also contain from 2 to 20% by weight of glycerine, propylene glycol or other humectant with a moisturising property. The lipid components of the composition preferably form a discontinuous phase in an aqueous emulsion containing and in which water comprises from 15 to 85% by weight of the emulsion.

The preferred anti-bacterial systems are sold under the trade marks "Myavert-C" and "Biovert". These systems are a mixture of natural enzymes, buffers and substrates that convert oxygen, which may be atmospheric, into oxidative compounds having anti-bacterial action. The main enzymes used are lactoperioxidase and glucose oxidase. Glucose is present as a substrate and the mixture is buffered to a pH in the range 5.0 to 6.5, preferably 5.3 to 5.6 The system is activated by admixing a combination of potassium iodide and sodium thiocyanate. The enzymes oxidase the iodide ions to iodate and the thiocyanate ions to hypothiocyanite. The oxidised ions selectively oxidise bacteria and fungal cell walls killing both bacteria and fungi.

The antibacterial action of the combination of these ingredients is described in WO-A-91/11105. Other enzyme based anti-bacterial systems, such as lactoferrin, are also effective in the control of bacteria and fungi in the compositions of the present invention.

It has been discovered that the combination of boswellic acids with the enzyme systems and also the anti-inflammatory agents based on polyunsaturated fatty acids and/or their esters e.g. linoleic acid or ethyl linolate with the compounds of the anti microbial agent e.g. glucose oxidase and lactoperoxidase, as used in the compositions of this invention, selectively slow down the cell turn over of SVK-14 cells (immortalised keratinocyte cell lines derived from human neonatal foreskin that are hyperproliferative) but has no effect on normal cells. This action overcomes psoriasis.

Well known moisturisers, emollients and humectants can be used as skin restorer. The preferred compounds are based on pure linoleic acid or plant derived oils that are rich in linoleic acid.

The compositions of this invention in one preferred form contain the anti-inflammatory agent, linoleic acid, and the enzyme based anti-microbial system in a pharmaceutically acceptable carrier.

Due to its unsaturated nature linoleic acid (pure or as part of plant oil) is not stable when exposed to air and is easily degraded, mainly by oxidation, to aldehyde and alcohol derivatives. To prevent such degradation the majority of commercially available plant oils and purified linoleic acid contain tocopherol (Vitamin E) as an antioxidant. The presence of tocopherol in the compositions of the invention has a detrimental effect on the ability of linoleic acid to slow down the cell turnover of keratinocyte cells. The linoleic acid used in the compositions of the invention should be substantially free from any antioxidants.

The enzyme based anti-microbial system (Myavert C) functions initially by reacting with the available oxygen to form peroxides which in turn are consumed by the lactoperoxidase to produce active species that destroy bacteria and fungi. The presence of the enzyme of the compositions of the invention appears to stablilise the linoleic acid since it removes oxygen and hence prevents the oxidation of linoleic acid.

The linoleic acid in the compositions of this invention performs five functions:
1. it acts as an anti-inflammatory agent;
2. it reduces selectively the cell turn over of hyperproliferative cells;
3. it softens the hard skin present;
4. it acts as a moisturiser and an emollient and
5. it helps to restore the stratum corneum to normal so that it can perform its barrier function (prevent water loss, ingress of chemicals and micro organisms).

The overall function of the enzyme based antibacterial system "Myavert C" in the composition of this invention is:
1. to remove oxygen from the enclosed system (e.g. a cream in a tube or jar) and hence stabilise the linoleic acid;
2. to act as a preservative to stabilise the product;
3. to destroy anti-microbials from the skin surface in use and
4. to reduce selectively the cell turn over of hyperproliferative cells.

The topical compositions of this invention are provided for use in a pharmaceutically acceptable carrier and this could take the form of a solution, cream, ointment, emulsion or balm or any other form well known in the art and the preferred carrier form is an oil in water emulsion cream.

The Table below lists the preferred components of the compositions/carriers according to this invention. Alternative carriers well known in the art and based on GRAS (generally regarded as safe) components could be substituted for the base given below.

**Function and Range of Components**

| **Component** | **Function** | **Range, % w/w** |
|---|---|---|
| water | carrier phase | 20-80 |
| Permulen TR2 NF (Carbomer) | emulsifier | up to 2 |
| Lactic Acid | pH buffer | up to 5 |
| Carbopol 980NF | thickener | up to 2 |
| Glycerine | humectant | up to 20 |
| Caprylic/capric triglycerides | Pharmaceutical solvent, solubiliser for Boswellia | up to 80 |
| Boswellia | Anti-inflammatory | up to 25 |
| Linoleic Acid | | up to 25 |
| Sunflower oil | emollient | up to 25 |
| Polyoxyethylene sorbitan monooleate | surfactant | up to 10 |
| 2,4-dichlorobenzyl alcohol | antifungal agent | up to 2 |
| Sodium lactate | pH buffer | up to 5 |
| Myavert C | Preservative and anti-bacterial agent | up tp 25 |

### Formulations

The following Table gives examples of formulations.

| Ingredients | 6A | 6B | 44 | 15 A6 | 15 B6 % w/w | 18A | 18B | 20A | 20B |
|---|---|---|---|---|---|---|---|---|---|
| Water I | 13.6 0 | 17.6 0 | | 13.2 0 | 13.2 0 | 24.5 5 | 28.5 5 | 5.15 | 5.88 |
| Lactic Acid (90%) | 0.25 | 0.25 | 0.25 | 0.25 | 0.25 | - | - | 0.20 | 0.24 |
| Citric Acid | - | - | - | - | - | 0.15 | 0.15 | - | - |
| Pemulen TR2 NF (3.0 % solution) | 13.7 0 | 13.7 0 | 13.7 0 | 15.0 0 | 15.0 0 | 13.7 0 | 13.7 0 | 15.0 0 | 17.6 6 |
| Carbopol 900 NF (2.0 % solution) | 20.4 0 | 20.4 0 | 20.4 0 | 17.5 0 | 17.5 0 | 20.4 0 | 20.4 0 | 15.0 0 | 17.6 6 |
| Glycerine | 5.00 | 5.00 | 5.00 | 5.00 | 5.00 | 4.00 | 4.00 | 4.00 | 4.71 |
| Caprylic Capric Triglycarides | 36.0 0 | 36.0 0 | 36.0 0 | 24.0 0 | 24.0 0 | 16.0 0 | 16.0 0 | 25.0 0 | 29.4 3 |
| Roswellia serata with total boswellic acid isomers | 4.00 | | 4.00 | - | - | 4.00 | - | 15.0 0 | - |
| Sunflower oil | - | - | - | - | 16.5 0 | 12.0 0 | 12.0 0 | 12.0 0 | 14.1 2 |
| Linoleic Acid-92.6 % active level (with - 50ppm nat vit E) | - | - | 20.0 0 | 16.5 0 | - | - | - | - | - |
| Surfacare T80 (Polyoxycthylene sorbitan monooleate) | 0.30 | 0.30 | 0.30 | 0.30 | 0.30 | 0.25 | 0.25 | 0.40 | 0.47 |
| Myacide SP | 0.15 | 0.15 | 0.15 | 0.15 | 0.15 | 0.15 | 0.15 | 0.15 | 0.15 |
| (2, 4-dichlorobenzyl] alcohol) Water II | 2.00 | 2.00 | | 2.00 | 2.00 | 2.00 | 2.00 | 2.00 | 2.35 |
| Sodium Lactate (60%) | 2.00 | 2.00 | 2.00 | 2.50 | 2.50 | | | 2.00 | 2.35 |
| Sodium citrate | - | - | - | - | - | 1.25 | 1.25 | - | - |
| TEA (50% sol.) | - | - | - | 1.00 | 1.00 | - | - | 1.50 | 2.30 |
| Myavert C part 1 | 2.00 | 2.00 | - | 2.00 | 2.00 | 1.00 | 1.00 | 2.00 | 2.00 |
| Water III | 0.50 | 0.50 | - | 0.50 | 0.50 | 0.50 | 0.50 | 0.50 | 0.59 |
| Myavert C part 2 | 0.10 | 0.10 | - | 0.10 | 0.10 | 0.05 | 0.05 | 0.10 | 0.10 |
| Total | 100. 00 | 100. 00 | 100. 00 | 100. 00 | 100. 00 | 100. 0 | 100. 00 | 100. 00 | 100. 00 |

### Typical Process of Preparation

The above formulations can be prepared using the Cold procedure described below.

| Stage | Action |
|---|---|
| 1 | Prepare a 3.0% Solution of Premulen TR2 NF as described in manufactuer's procedure |
| 2 | Prepare a 2.0% Solution of Carobpol 980 NF as described in the manufacturer's procedure |
| 3 | Mix Boswellic Acid and Caprylic/Capric Triglycerides and heat to 115-120°C. Hold at this temperature for 30 minutes and then cool room temperature. The temperature of the mix must be maintained between 22-24° C throughout this procedure4 |
| 4 | In a suitable mixing vessel with an anchor stirrer and homogeniser add Lactic Acid and water 1 and stir for 5 minutes. |
| 5 | Add Premulen TR2 NF solution from stage 1 and stir for 20 minutes. |
| 6 | Add Carbomer 908 NF solution from stage 2 and stir for 20 minutes. |
| 7 | Add glycerine, stir for 20 minutes. |
| | (WATER PHASE) |
| 8 | To-Boswellia serrata and Caprylic/Capric Triglycerides solution of stage 3 at 22-24°C add linoleic acid and/or sunflower oil, polyoxyethylene sorbitan monooleate and 2,4-dichlorobenzyl alcohol and stir for 30 minutes (OIL PHASE) |
| 9 | Ensure oil phase is about 2°C above the temperature of water phase. |
| 10 | Add oil phase to water phase with rapid stirring over 5 minutes and continue mixing for 15 minutes to form an oil in water emulsion cream. |
| 11 | Cool the emulsion to contain temperature between 22-24°C if necessary. |
| 12 | Dissolve sodium citrate in water 2 and add to the main emulsion mix with rapid stirring; stir for 5 minutes. |
| 13 | Check pH |
| 14 | Slow the stirrer to gentle mixing and adjust pH with TEA (50%) to 5.4-5.6 at and stir for 20 minutes. |
| 15 | Add Myavert C part 1 and stir for 15 minutes. |
| 16 | Dissolve Myavert C part 2 into water 3 and add to the emulsion and stir for 15 minutes. |
| 17 | Check and record final pH and viscosity of the emulsion (Specification pH=5.4-5.6 and Viscosity = 20,0000-80,000 cps) |

The Boswellia serrata containing compositions can also be prepared using the HOT procedure described below.

| Stage | Action |
|---|---|
| 1 | Prepare a 3.0% solution of Premulen TR2 NF as described in manufacturer's procedure |
| 2 | Prepare a 2.0% solution of Carbopol 980 NF as described in manufacturer's procedure |
| 3 | Mix Boswellic acids(s) and Caprylic/Capric Triglycerides and heat to 115-120°C. Hold at this temperature for 30 minutes and then cool 82-84°C. |
| 4 | In a suitable mixing vessel with an anchor stirrer and homogeniser add Lactic Acid and water 1 and stir for 5 minutes |
| 5 | Add Premulen TR2 NF solution from stage 1 and stir for 20 minutes |
| 6 | Add Carbomer 980 NF solution from stage 2 and stir for 20 minutes |
| 7 | Add glycerine, stir for 20 minutes then raise the temperature to 80-82°C (WATER PHASE) |
| 8 | To Boswellic acid(s) and Caprylic/Capric Triglycerides solution of stage 3 at 82-84°C add linoleic acid and/or sunflower oil, polyoxyethylene sorbitan monooleate and 2,4-dichlorobenzyl alcohol and stir for 30 minutes (OIL PHASE) |
| 9 | Ensure oil phase is about 2° C above the temperature of water phase |
| 10 | Add oil phase to water phase with rapid stirring over 5 minutes and continue mixing for 15 minutes to form an oil in water emulsion cream |
| 11 | Start cooling the emulsion |
| 12 | Dissolve sodium citrate in water 2 and add to the main emulsion mix with rapid stirring at 45-50°CV; stir for 5 minutes |
| 13 | Check pH |
| 14 | Slow the stirrer to gentle mixing and adjust pH with TEA (50%) to 5.4-5.6 at 40-45°C and stir for 20 minutes |
| 15 | Add myavert to C part 1 and stir for 15 minutes |
| 16 | Dissolve Myavert C part 2 into water 3 and add to the emulsion and stir for 15 minutes |
| 17 | Check and record final pH and viscosity of the emulsion (Specification pH=5.4-5.6 and Viscosity =.20,000-80,000 cps) |

### CLINICAL TRIALS

### Formulations 18A and 19B

A male child aged 2 1/2 years (Patient No. 1/98) had atopic eczema covering more than 18% of the body surface. The eczema had been present for two weeks and left untreated. There was visual indication of bacterial infection. Eczema Cream 18A was applied to the affected areas of the patient on a regular basis. After 3 days the redness and irritation had subsided. After two months the pruritis was eliminated and all the symptoms of eczema were overcome within 2 1/2 months, restoring the skin to its normal health. The patient remained in remission for at least three years. (This was the last point at which he was available for examination.)

22 patients were treated with cream 18A and 22 with cream 18B.. Both creams overcame eczema on patients and 18A gave a faster response on average overcoming eczema in two weeks whereas 18B took 3 to 4 weeks. Patients treated with 18A showed a lower relapse rate than those treated with 18B.

### Clinical results of Pilot study on the effects of 15A and 15B cream formulations in the treatment of Psoriasis

A clinical trial was carried out using selected creams (**15A** -15% of 94% = 14.1%) active linoicic acid and enzyme based anti-bacterial system versus the (15B) enzyme based anti-bacterial system only in an emollient base, to establish the overall efficacy of the optimized formulations and their ability to control psoriasis.

Information was recorded in questionnaire, and where possible photographic, form. During the first consultation with patients one of two topical cream treatments, named either 15A or 15B, was prescribed with instructions to apply the cream twice a day up to two weeks after clearance of lesions. **15A** is Linoleic acid (14.1%) and enzyme based anti-bacterial system in an emollient base. **15B** is an enzyme based anti-bacterial system in an emollient base only. Progression in clinical state was recorded in questionnaire, note and photographic form, as and when the patients returned to the clinic.

Two male and four female participants were recruited from patients diagnosed with psoriasis and confirmed by biopsy attending a Dermatology Clinic. A wide range of participants (N) were recruited. Age and prior duration of the participants' psoriasis condition was recorded in years a percentage of the body area covered with psoriasis. The data was coded as; (A) 1% or less, (B) 1-5%, (C) 10%, (D) 10-18% and (E) greater than 18%. The 'Types of Psoriasis' were recorded as Plaque, Erythrodermic, Guttate (drop), Inverse, Pustular, Scalp, Nail, Psoriatic Arthritis, others to be specified) at first visit and or following biopsy. A 'Body map' was marked by circling the effected area and 'Site of Lesion', was specified and coded as, (0) OK, (1) Mild, (2) Moderate, (3) Moderate to Severe, and (4) Severe. Lesions were also recorded in photographic form where the patient consented. The presence of fungal infection to visual examination was noted as either 'Yes', 'No' or 'Do not know', The 'Site of lesion' coding was also used to score the characteristics 'Exfoliation/Scalines' 'Pain, 'Redness and irritation', 'Pruritus', and 'Pustules', Detailed demographic data collected for each of the participants. The measure of response to treatment was recorded as the number of days taken for all lesions on all parts of the body to clear named, 'Cleared'. Improvement, clearance, and remission data is presented in the Table below.

**Improvement and Clearance For Participants Treated with 15A and 15B**

| PARTICIPANTS TREATED WITH 15A | | | | | | |
|---|---|---|---|---|---|---|
| Participant Number | Improvement seen at | Cleared | Remission after clearance | | Total | Last recor ded visit |
| | Day | Nos of days | Nos of days | Nos. of visits | Nos. of visits | Nos. of days |
| 1 | 21, 35, 63, 91, 119 | 147 | 51 | 2 | 9 | 198 |
| 3 | 4 and 72 | 72 | - | - | 3 | 72 |
| 4 | 7, 14, and 44 | 44 | - | - | 4 | 44 |
| 5 | 14 | 30 | - | - | 3 | 30 |
| 6 | 24 | 56 | - | - | 3 | 47 |

| PARTICIPANTS TREATED WITH 15B | | | | | | |
|---|---|---|---|---|---|---|
| 2 | 24 and 40 | 40 | - | - | 3 | 40 |

## Claims

1. Use of a composition comprising, in combination, one or more boswellic acids and an enzyme based antibacterial system comprising lactoperoxidase and glucose oxidase in the manufacture of a medicament for the treatment of eczema and psoriasis.

2. Use of a composition comprising, in combination, a fatty acid having a chain length of at least 18 carbon atoms and which contains at least two unsaturated linkages and an enzyme based antibacterial system comprising lactoperoxidase and glucose oxidase in the manufacture of a medicament for the treatment of psoriasis.

3. Use of a composition comprising, in combination, one or more boswellic acids, an enzyme based antibacterial system comprising lactoperoxidase and glucose oxidase and a fatty acid having a chain length of at least 18 carbon atoms and which contains at least two unsaturated linkages in the manufacture of a medicament for the treatment of eczema and psoriasis.

4. Use of a composition as claimed in any one of the preceding claims wherein glucose is present as a substrate and the mixture is buffered to a pH in the range 5 to 6.5, preferably to a PH in the range 5.3 to 5.6.

5. Use of a composition as claimed in any one of the preceding claims, in which the enzyme based antibacterial system is activated by admixture with a combination of potassium iodide and sodium thiocyanate.

6. Use of a composition as claimed in any one of the preceding claims in which a fatty acid or a combination of fatty acids either saturated or unsaturated and containing 8 to 28 carbon atoms is present as a delivery agent.

7. Use of a composition as claimed in any one of the preceding claims in the form of a cream emulsion for topical application containing from 5 to 40% by weight of sunflower, safflower, soyabean, corn, evening primrose, refined herring or tuna oil and any other oil as carrier.

8. Use of a composition as claimed in any one of the preceding claims, containing from 10 to 80% by weight of caprylic/capric triglycerides.

9. Use of a composition as claimed in any of the preceding claims, containing from 2 to 20% by weight of glycerine, propylene glycol and any other humectant with a moisturizing property.

10. Use of a composition as claimed in claims 7 to 10, in which the lipid components form a discontinuous phase in an aqueous emulsion in which water comprises from 15 to 85% by weight of the emulsion.

11. A composition comprising, in combination, one or more boswellic acids and an enzyme based antibacterial system comprising lactoperoxidase and glucose oxidase for use in the treatment of eczema and psoriasis

12. A composition comprising, in combination, a fatty acid having a chain length of at least 18 carbon atoms and which contains at least two unsaturated linkages and an enzyme based antibacterial system comprising lactoperoxidase and glucose oxidase for use in the treatment of psoriasis.

13. A composition comprising, in combination, one or more boswellic acids, an enzyme based antibacterial system comprising lactoperoxidase and glucose oxidase and a fatty acid having a chain length of at least 18 carbon atoms and which contains at least two unsaturated linkages for use in the treatment of eczema and psoriasis.

## Patentansprüche

1. Verwendung einer Zusammensetzung umfassend, in Kombination, eine oder mehrere Boswelliasäuren und an enzymbasiertes antibakterielles System umfassend Laktoperoxidase und Glukoseoxidase in der Herstellung eines Medikaments für die Behandlung von Ekzem und Psoriasis.

2. Verwendung einer Zusammensetzung umfassend, in Kombination, eine Fettsäure mit einer Kettenlänge von mindestens 18 Kohlenstoffatomen, und die mindestens zwei ungesättigte Bindungen und ein enzymbasiertes antibakterielles System umfassend Laktoperoxidase und Glukoseoxidase enthält, in der Herstellung eines Medikaments für die Behandlung von Psoriasis.

3. Verwendung einer Zusammensetzung umfassend, in Kombination, eine oder mehrere Boswelliasäuren, ein enzymbasiertes antibakterielles System umfassend Laktoperoxidase und Glukoseoxidase und eine Fettsäure mit einer Kettenlänge von mindestens 18 Kohlenstoffatome, und die mindestens zwei ungesättigte Bindungen enthält, in der Herstellung eines Medikaments für die Behandlung von Ekzem und Psoriasis.

4. Verwendung einer Zusammensetzung nach einem der vorherigen Ansprüche, wobei Glukose als ein Substrat vorhanden ist und das Gemisch auf einen pH im Bereich 5 bis 6,5, vorzugsweise auf einen PH im Bereich 5,3 bis 5,6 gepuffert ist.

5. Verwendung einer Zusammensetzung nach einem der vorherigen Ansprüche, wobei das enzymbasierte antibakterielle System durch Beimischung mit einer Kombination aus Kaliumiodid und Natriumthiocyanat aktiviert wird.

6. Verwendung einer Zusammensetzung nach einem der vorherigen Ansprüche, wobei eine Fettsäure oder eine Kombination von Fettsäuren entweder gesättigt oder ungesättigt und enthaltend 8 bis 28 Kohlenstoffatome als ein Zuführungsmittel verwendet wird.

7. Verwendung einer Zusammensetzung nach einem der vorherigen Ansprüche in Form einer Creme-Emulsion für topische Anwendung enthaltend 5 bis 40 Gew.-% Sonnenblumen-, Saflor-, Sojabohnen-, Mais-, Nachtkerzen-, raffiniertes Herings- oder Thunfischöl und jedes beliebige andere Öl als Träger.

8. Verwendung einer Zusammensetzung nach einem der vorherigen Ansprüche, enthaltend 10 bis 80 Gew.-% Caprylin-/Caprintriglyceride.

9. Verwendung einer Zusammensetzung nach einem der vorherigen Ansprüche, enthaltend von 2 bis 20 Gew.-% Glycerin, Propylenglycol und jedes andere Befeuchtungsmittel mit einer feuchtigkeitsspendenden Eigenschaft.

10. Verwendung einer Zusammensetzung nach einem der Ansprüche 7 bis 10, wobei die Lipid-Komponenten eine diskontinuierliche Phase in einer wässrigen Emulsion bilden, wobei Wasser 15 bis 85 Gew.-% der Emulsion umfasst.

11. Zusammensetzung, umfassend, in Kombination, eine oder mehrere Boswelliasäuren und an enzymbasiertes antibakterielles System umfassend Laktoperoxidase und Glukoseoxidase zur Verwendung in der Behandlung von Ekzem und Psoriasis

12. Zusammensetzung, umfassend, in Kombination, eine Fettsäure mit einer Kettenlänge von mindestens 18 Kohlenstoffatomen, und die mindestens zwei ungesättigte Bindungen und ein enzymbasiertes antibakterielles System umfassend Laktoperoxidase und Glukoseoxidase enthält, zur Verwendung in der Behandlung von Psoriasis.

13. Zusammensetzung, umfassend, in Kombination, eine oder mehrere Boswelliasäuren, ein enzymbasiertes antibakterielles System umfassend Laktoperoxidase und Glukoseoxidase und eine Fettsäure mit einer Kettenlänge von mindestens 18 Kohlenstoffatomen, und die mindestens zwei ungesättigte Bindungen enthält, zur Verwendung in der Behandlung von Ekzem und Psoriasis.

## Revendications

1. Utilisation d'une composition comprenant, en combinaison, un ou plusieurs acides boswelliques et un système antibactérien aux enzymes comprenant la lactoperoxydase et la glucose-oxydase dans la fabrication d'un médicament pour le traitement de l'eczéma et du psoriasis.

2. Utilisation d'une composition comprenant, en combinaison, un acide gras ayant une longueur de chaîne d'au moins 18 atomes de carbone et qui contient au moins deux liaisons non saturées et un système antibactérien aux enzymes comprenant la lactoperoxydase et la glucose-oxydase dans la fabrication d'un médicament pour le traitement du psoriasis.

3. Utilisation d'une composition comprenant, en combinaison, un ou plusieurs acides boswelliques, un système antibactérien aux enzymes comprenant la lactoperoxydase et la glucose-oxydase et un acide gras ayant une longueur de chaîne d'au moins 18 atomes de carbone et qui contient au moins deux liaisons non saturées dans la fabrication d'un médicament pour le traitement de l'eczéma et du psoriasis.

4. Utilisation d'une composition selon l'une quelconque des revendications précédentes, dans laquelle le glucose est présent en tant que substrat et le mélange est tamponné à un pH dans la gamme de 5 à 6,5, de préférence à un pH dans la gamme de 5,3 à 5,6.

5. Utilisation d'une composition selon l'une quelconque des revendications précédentes, dans laquelle le système antibactérien aux enzymes est activé par mélange avec une combinaison d'iodure de potassium et de thiocyanate de sodium.

6. Utilisation d'une composition selon l'une quelconque des revendications précédentes, dans laquelle un acide gras ou une combinaison d'acides gras soit saturés soit non saturés et contenant 8 à 28 atomes de carbone est présent comme agent d'administration.

7. Utilisation d'une composition selon l'une quelconque des revendications précédentes sous la forme d'une émulsion crémeuse pour application topique contenant de 5 à 40 % en poids d'huile de tournesol, de carthame, de soja, de maïs, d'onagre, de hareng ou de thon raffinée et de toute autre huile comme excipient.

8. Utilisation d'une composition selon l'une quelconque des revendications précédentes, contenant de 10 à 80 % en poids de triglycérides capryliques/capriques.

9. Utilisation d'une composition selon l'une quelconque des revendications précédentes, contenant de 2 à 20% en poids de glycérine, propylène glycol et tout autre humectant ayant une propriété hydratante.

10. Utilisation d'une composition selon les revendications 7 à 10, dans laquelle les composants lipides forment une phase discontinue dans une émulsion aqueuse dans laquelle l'eau constitue de 15 à 85 % en poids de l'émulsion.

11. Composition comprenant, en combinaison, un ou plusieurs acides boswelliques et un système antibactérien aux enzymes comprenant la lactoperoxydase et la glucose-oxydase pour utilisation dans le traitement de l'eczéma et du psoriasis

12. Composition comprenant, en combinaison, un acide gras ayant une longueur de chaîne d' au moins 18 atomes de carbone et qui contient au moins deux liaisons non saturées et un système antibactérien aux enzymes comprenant la lactoperoxydase et la glucose-oxydase pour utilisation dans le traitement du psoriasis.

13. Composition comprenant, en combinaison, un ou plusieurs acides boswelliques, un système antibactérien aux enzymes comprenant la lactoperoxydase et la glucose-oxydase et un acide gras ayant une longueur de chaîne d'au moins 18 atomes de carbone et qui contient au moins deux liaisons non saturées pour utilisation dans le traitement de l'eczéma et du psoriasis.
